# EUROPEAN PATENT SPECIFICATION

(11) **EP 3 573 645 B1**
(45) Date of publication and mention of the grant of the patent: **13.03.2024**
(21) Application number: 18748798.8
(22) Date of filing: 01.02.2018
(51) Int. Cl.: A61K 38/08, C07K 14/68, C07K 14/685, A61P 17/00, A61K 38/10, A61K 38/12, A61K 38/34

(54) **ALPHA-MSH ANALOGUES USED IN THE TREATMENT OF XERODERMA PIGMENTOSUM**
ALPHA-MSH-ANALOGA ZUR BEHANDLUNG VON XERODERMA PIGMENTOSUM
ANALOGUES D'ALPHA-MSH UTILISÉS DANS LE TRAITEMENT DU XERODERMA PIGMENTOSUM

(30) Priority: 01.02.2017 EP 17154272
(43) Date of publication of application: 04.12.2019
(73) Proprietor: VALLAURIX MC S.A.R.L., 98000 Monaco (MC)
(72) Inventor: WOLGEN, Philippe, Melbourne, Victoria 3000 (AU)
(74) Representative: Farago-Schauer, Peter Andreas
(86) International application number: PCT/IB2018/050629
(87) International publication number: WO 2018/142318

(56) References cited:
- EP-A1- 3 088 418
- WO-A1-2007/123699
- WO-A1-2008/025094
- WO-A1-2010/052253
- WO-A1-2011/063366
- WO-A1-2011/063367
- WO-A1-2012/131090
- WO-A1-2012/131090
- WO-A1-2013/001030
- WO-A1-2016/174610
- US-A1- 2011 275 657
- LIANG DONG ET AL: "Melanocyte-stimulating hormone directly enhances UV-Induced DNA repair in keratinocytes by a xeroderma pigmentosum group A-dependent mechanism", CANCER RESEARCH, AACR, US PHILADELPHIA, PA, vol. 70, no. 9, May 2010 (2010-05), pages 3547-3556, XP002677495, ISSN: 1538-7445, DOI: 10.1158/0008-5472.CAN-09-4596 [retrieved on 2010-04-13]
- ZALFA A. ABDEL-MALEK ET AL: "[alpha]-MSH tripeptide analogs activate the melanocortin 1 receptor and reduce UV-induced DNA damage in human melanocytes", PIGMENT CELL & MELANOMA RESEARCH, vol. 22, no. 5, 1 October 2009 (2009-10-01), pages 635-644, XP055533945, United States, Denmark ISSN: 1755-1471, DOI: 10.1111/j.1755-148X.2009.00598.x
- ADBEL-MALEK, Z. A. et al.: "a-MSH tripeptide analogs activate the melanocortin 1 receptor and reduce UV-induced DNA damage in human melanocytes", Pigment Cell Melanoma Res., vol. 22, 2009, pages 635-644, XP055533945,
- DONG, L. et al.: "Melanocyte-Stimulating Hormone Directly Enhances UV-Induced DNA Repair in Keratinocytes by a Xeroderma Pigmentosum Group A-Dependent Mechanism", Cancer Research, vol. 70, no. 9, 2010, pages 3547-3556, XP002677495,

## Description

### Technical field

The present invention is directed to compounds for use in treatment of a human subject suffering from a particular medical indication.

### Background to the invention

Xeroderma Pigmentosum (XP) is an autosomal recessive genetic disorder in which repair of DNA (deoxyribonucleic acid) is deficient. There is a clinical need for improvements of treatment of XP and of reducing its symptoms and improving the quality of life of XP subjects.

### Summary of the invention

We have found surprising clinical benefits of alpha-MSH analogue compounds in treatment of Xeroderma Pigmentosum (XP). Accordingly, the present invention relates to an alpha-MSH analogue compound for use in enhancing DNA repair in a subject suffering from Xeroderma Pigmentosum (XP). More generally, the invention relates to an alpha-MSH analogue compound for use in the treatment of a human subject suffering from Xeroderma Pigmentosum (XP), wherein the compound has agonist activity for the melanocortin-1-receptor (MC1R) receptor. Specifically, the present invention is directed to use in enhancing light-induced DNA repair, in particular Ultra Violet (UV)-induced DNA repair.

In an embodiment of the invention, the alpha-MSH analogue compound has formula structure: Ac - Nle - Glu - His- D-Phe - X - Trp - NH₂,
wherein X is homoArg or norArg, or a pharmaceutically acceptable salt thereof.

In another embodiment of the invention, the alpha-MSH analogue compound has formula structure:
R₁ R₂ R₃ N - (CH2)ₙ - CO - Nle - Glu - His- D-Phe - X - Trp - NH₂ wherein:
R₁, R₂ and R₃ are independently selected from methyl, ethyl, and propyl;
n is from 1-4; and
X is selected from Arg, norArg and homoArg,
or a pharmaceutically acceptable salt thereof.

In another embodiment of the invention, the alpha-MSH analogue compound is [Nle⁴, D-Phe⁷]-alpha-MSH, or a pharmaceutically acceptable salt thereof.

Preferably, the XP disorder is selected from complementation group A (XP-A), complementation group B (XP-B), complementation group C (XP-C), and complementation group E (XPE) and F (XP-F) and variant type V (XPV). Generally, the XP disorder is selected from complementation group A (XP-A), complementation group B (XP-B), complementation group C (XP-C), complementation group D (XP-D), complementation group E (XP-E), complementation group F (XP-F), complementation group G (XP-G), and variant type (XP-V).

Preferably, the alpha-MSH analogue compound is administered in a composition providing plasma levels of the compound of less than 10ng/ml for at least 1 day. Preferably, the alpha-MSH analogue compound is administered in the form of an extended release composition, preferably a subcutaneous implant. Preferably, the composition comprises from 4 to 20mg of the compound. Preferably, the composition is administered with a dosing frequency of between 5 to 15 days. Preferably, the composition is administered at least 3 times consecutively to the subject.

The alpha-MSH analogue of the invention is typically used in a method of enhancing DNA repair in a subject suffering from Xeroderma Pigmentosum (XP) by administering the alpha-MSH analogue with agonist activity for the MC1R receptor to a subject. Generally, the alpha-MSH analogue of the invention is used in a method of treating Xeroderma Pigmentosum (XP) by administering an alpha-MSH analogue compound with agonist activity for the MC1R receptor to a subject.

Preferably, the alpha-MSH analogue compound is administered to a human subject with an interval between subsequent administrations of the compound of between 5 to 15 days. Further, the alpha-MSH analogue of the invention is used in the manufacture of a medicament for enhancing DNA repair in a subject suffering from Xeroderma Pigmentosum (XP).

We have surprisingly found that the invention provide for effective yet safe and convenient treatment of XP using alpha-MSH analogues, preferably at least partially reducing one or more of the symptoms associated with XP, and, specifically, the invention is directed to enhancing UV-induced DNA repair.

### Details description of the invention

The references to the methods of treatment by therapy or surgery of this description are to be interpreted as references to compounds, pharmaceutical compositions and medicaments of the present invention for use in those method.

For the purpose of this invention, treatment is defined as encompassing prevention of a disorder. Further, treatment is defined as encompassing reduction of symptoms associated with the disease.

For the purpose of this invention, the following nucleic acids abbreviations are used: Ala = alanine, Arg = arginine, Dab - 2,4-diaminobutyric acid, Dpr = 2,3- diaminopropionic acid, Glu = glutamic acid, Gly = glycine, His = histidine, HomoArg - homoarginine (one more -CH₂- unit in the alkyl chain than Arg), norArg - norarginine (one fewer -CH2 unit in the alkyl chain than Arg), Lys = lysine, Met = methionine, Nle = norleucine, Orn = ornithine, Phe = phenylalanine, (pNO2)Phe = paranitrophenylalanine, Plg = phenylglycine, Pro = proline, Ser = serine, Trp = tryptophan, TrpFor = N¹ formyl-tryptophan, Tyr = tyrosine, Val = valine.

For the purpose of this invention, all peptides are written with the acyl-terminal end at the left and the amino terminal end to the right; the prefix "D" before an amino acid designates the D-isomer configuration, and unless specifically designated otherwise, all amino acids are in the L-isomer configuration. All peptide and peptide derivatives are written with the acylated amino terminal end at the left and the amidated carboxyl terminal at the right. As will be understood, the acylated amino terminal end may be replaced by another group according to the invention but the orientation of the peptides and peptide derivatives remains the same. Following common convention, the first amino acid on the left is located at position 1, for instance, Nle (1) indicating that Nle is positioned at the N terminal end (on the left). HomoArg and norArg may be referred to as amino acids even though they are strictly amino acid derivatives. In the same way, compounds comprising quaternary ammonium groups, homoArg, norArg and/or other amino acid derivatives may be referred to as peptides even though they are strictly peptide derivatives. Accordingly, the skilled person will understand that reference in this document to peptide molecules (such as hexapeptides or alpha-MSH analogues) includes reference to derivatives thereof.

For the purpose of this invention, synthetic is defined as not being naturally present in animals but instead being human-prepared. For instance, the alpha-MSH hormone falls outside the definition of synthetic.

For the purpose of this invention, the alpha-MSH analogues may be used as such or in the form of a pharmaceutically acceptable salt thereof. Preferred examples of such salts are acetate, trifluoroacetate, sulfate, and chloride salts. The acetate salt is generally most preferred.

For the purpose of this invention, UV light is defined as light with a wavelength of 10-400nm. The invention is in particular directed to DNA damage induced by UV light comprising UV-A (315-400nm), UV-B (280-315nm) and UV-C (100-280nm).

According to the invention, we have surprisingly found that alpha-MSH analogues are effective in treatment of human subjects suffering from Xeroderma Pigmentosum (XP). For the purpose of this invention, the term Xeroderma Pigmentosum refers to an autosomal recessive, genetic disorder of human subjects characterized by an extreme sensitivity to UV rays. Symptoms of XP usually occur at infancy or early childhood and include freckling of the sun-exposed skin, dry skin, and changes in pigmentation. Subjects suffering from XP are particularly susceptible to increased risk of various cancer types and neurological abnormalities. Without wishing to be bound by any theory, it is believed that the ability to repair DNA damage in XP subjects is compromised and/or at least partially disabled.

At least eight inherited forms of XP have been identified based on genes, including complementation group A (XP-A), complementation group B (XP-B), complementation group C (XP-C), complementation group D (XP-D), complementation group E (XP-E), complementation group F (XP-F), complementation group G (XP-G), and variant type (XP-V).

Accordingly, the invention relates to treatment of complementation group A (XP-A). The invention further relates to treatment of complementation group B (XP-B). The invention further relates to treatment of complementation group C (XP-C). The invention further relates to treatment of complementation group D (XP-D). The invention further relates to treatment of complementation group E (XP-E). The invention further relates to treatment of complementation group F (XP-F). The invention further relates to treatment of complementation group G (XP-G). And the invention further relates to treatment of variant type (XP-V). In particularly, the invention is preferably directed to treatment of complementation group A (XP-A), complementation group B (XP-B), complementation group C (XP-C) and complementation group F (XP-F).

The present invention relates to the use of alpha-MSH analogue compounds, wherein the compound preferably has agonist activity for the MC1R receptor. Different alpha-MSH analogue compounds have been described in the art and have been proposed for various purposes. For instance, WO2016/066700 discusses the use of low plasma levels of alpha-MSH analogues. WO2012/107592 discloses alpha-MSH analogues for photoprotection of the skin and enhancing repair of DNA damage in normal mammals. WO2009/118191 discusses the use of alpha-MSH analogues in vitiligo. WO2008/025074 relates to the use of alpha-MSH analogues in immunocompromised subjects. WO2008/025094 describes the use of alpha-MSH analogues in patients suffering from photodermatoses and does not refer to DNA repair. WO2005/048967 is directed to use of alpha-MSH analogues in subjects with MC1R variants. WO2012/131090 only theoretically proposes (and does not practice or exemplify) tanning XP patients with MC1R agonists in combination with light. Despite sunscreen use, such light exposes XP patients to additional UV, actually increasing DNA damage. WO2007/123699, WO2011/063366, and WO2011/063367 solely disclose the pigmentation effects of alpha-MSH analogues. US2011/0275657 relates to pigmentation through mC1R receptors in the melanocytes. WO2010/052253 only relates to melanin synthesis in the melanocytes. WO2013/001030 relates to pigmentation disorders. Liang Dong, et al's article on "Melanocyte-stimulating hormone directly enhances UV-induced DNA repair in keratinocytes by a xeroderma pigmentosum group A-dependent mechanism" (see Cancer research, AACR, US Philadelphia, PA, vol. 70, No. 9, May 2010 (2010-05), pages 3547-3556) discusses the effect of alpha-MSH on DNA repair in healthy subjects. The discussion mentions that [Nle⁴, D-Phe⁷]-alpha MSH is a pigment inducing molecule that has sunless tanning and sun damage reducing capabilities.

Zalfa A., Abdel-Malek et al's article on "alpha-MSH tripeptide analogs activate the melanocortin 1 receptor and reduce UV-induced DNA damage" (Pigment & melanoma research, Vol 22, No 5, 1 Oct 1999, page 635-644) discusses the effect of tripeptides on UV-induced DNA damage generally. EP308818 discloses homo- and nor-Arg alpha-MSH analogue hexapeptides for use in skin disorders.

It is preferred according to the present invention to administer the alpha-MSH analogue compound of the invention systemically. Preferably, the alpha-MSH analogue compound is administered by transdermal or cutaneous administration, and more preferably by subcutaneous administration. Preferred systemic administration of the alpha-MSH analogue of the invention is by way of an injection, more preferably by way of a subcutaneously injected implant. Preferred systemic administration is by way of a controlled-release composition, as defined below.

According to the invention, the subject suffering from XP is preferably exposed to alpha-MSH analogue at a blood plasma level of at least 0.01ng/ml, more preferably at least 0.1ng/ml, most preferably at least 1ng/ml and preferably at most 20ng/ml, more preferably at most 15ng/ml, most preferably at most 10ng/ml. Preferably, exposure of the XP subject to the alpha-MSH analogue of the composition of the invention is for at least 1 day, more preferably at least 2 days, more preferably at least 5 days. Preferably, the XP subject is subsequently continuously exposed to the alpha-MSH analogue, more preferably up to 1 year, or up to 6 months. Preferably, plasma levels are maintained for at least 10, 20, 30, 40, 50, or 60 days. In one embodiment, the XP subject is exposed for preferably at most 60 days, more preferably at most 40 days, most preferably at most 20 days and particularly preferred for at most 10 days, for instance up to 7 days or up to 10 days. Preferably, these alpha-MSH analogue blood plasma levels are achieved subsequent to -preferably within 1 day of- alpha-MSH analogue administration. According to a preferred treatment of the invention, the alpha-MSH analogue is administered at least 2 times to the subject, more preferably at least 3 times, most preferably at least 5 times and for instance up to 20 times, each composition providing the above mentioned exposure. Preferably, the dosing is consecutively. In a preferred embodiment, an subcutaneous afamelanotide implant is administered every 5 days to maintain plasm levels not exceeding 10ng/ml in the subject. A preferred implant has a 16mg strength. As will be understood by a skilled person in the art, after initial alpha-MSH analogue release from the drug composition and absorption by the subject into the blood plasma, the alpha-MSH analogue will be present in the blood plasma of the subject at the level and the time period indicated. Subsequently, the next dose is administered. Dose levels, plasma levels and dose frequencies may vary and are each preferably -independently- within the ranges for exposure given above. Thus, the alpha-MSH analogue is administered in a composition and in an amount that results in the blood plasma levels over time as indicated. Accordingly, the human subject is subjected to the blood plasma levels indicated. It will be understood that for the purpose of the invention, intervals are separate and subsequent and do not overlap.

According to one aspect, the invention is directed to alpha-MSH analogue compounds. The term "alpha-MSH analogue" as used herein is defined as a derivative of alpha-MSH which exhibits agonist activity for the melanocortin-1-receptor (MC1R), the receptor to which alpha-MSH binds to initiate the production of melanin within a melanocyte. Preferably, the alpha-MSH analogue compound of the invention is a synthetic molecule. Such alpha-MSH analogues include derivatives in which (i) one or more amino acid residues are deleted from the native alpha-MSH molecule at the N-terminal end, the C-terminal end, or both; and/or (ii) one or more amino acid residues of the native alpha-MSH molecule are replaced by another natural, non-natural or synthetic amino acid residue; and/or (iii) an intra-molecular interaction forms as a cyclic derivative. Several derivatives of alpha-MSH have been synthesized. In one aspect of the present invention, the alpha-MSH analogues described in US Patents Nos. 4,457,864, 4,485,039, 4,866,038, 4,918,055, 5,049,547, 5,674,839 and 5,714,576 and Australian Patents Nos. 597630 and 618733 can be used herein.

In one aspect of the invention, the alpha-MSH analogue is selected from the group consisting of:
(a) compounds of the formula:
   Ac-Ser-Tyr-Ser-M-Gln-His-D-Phe-Arg-Trp-Gly-Lys-Pro-Val-NH₂
   wherein M is Met, Nle or Lys; and
(b) compounds of the formula:
   R₁-W-X-Y-Z-R₂
   wherein
   R₁ is absent, n-pentadecanoyl, Ac, 4-phenylbutyryl, Ac-Gly-, Ac-Met-Glu, Ac-Nle-Glu-, or Ac-Tyr-Glu-;
   W is -His- or-D-His-;
   X is -Phe-, -D-Phe-, -Tyr-, -D-Tyr-, or -(pN0₂)D-Phe⁷-;
   Y is -Arg- or -D-Arg-;
   Z is -Trp- or -D-Trp-; and
   R₂ is -NH₂; -Gly-NH₂; or-Gly-Lys-NH₂, as disclosed in Australian Patent No. 597630.

In another aspect, the alpha-MSH analogue may be a linear analogue as disclosed in US 5674,839, and selected from the group consisting of:
Ac-Ser-Tyr-Ser-Nle-Glu-His-D-Phe-Arg-Trp-Lys-Gly-Pro-Val-NH₂,
Ac-Ser-Tyr-Ser-Nle-Asp-His-D-Phe-Arg-Trp-Lys-Gly-Pro-Val-NH₂,
Ac-Nle-Glu-His-D-Phe-Arg-Trp-Lys-Gly-Pro-Val-NH₂,
Ac-Nle-Asp-His-D-Phe-Arg-Trp-Lys-Gly-Pro-Val-NH₂,
Ac-Nle-Asp-His-D-Phe-Arg-Trp-Gly-NH₂,
Ac-Nle-Glu-His-D-Phe-Arg-Trp-Lys-NH₂,
Ac-Nle-Asp-His-D-Phe-Arg-Trp-Lys-NH₂,
Ac-Nle-Glu-His-D-Phe-Arg-Trp-Orn-NH₂,
Ac-Nle-Asp-His-D-Phe-Arg-Trp-Orn-NH₂,
Ac-Nle-Glu-His-D-Phe-Arg-Trp-Dab-NH₂,
Ac-Nle-Asp-His-D-Phe-Arg-Trp-Dab-NH₂,
Ac-Nle-Glu-His-D-Phe-Arg-Trp-Dpr-NH₂,
Ac-Nle-Glu-His-Phe-Arg-Trp-Lys-NH₂, and
Ac-Nle-Asp-His-Phe-Arg-Trp-Lys-NH₂.

In another aspect, the alpha-MSH analogue may also be a cyclic analogue as disclosed in US Patent No. 5,674,839, selected from the group consisting of:

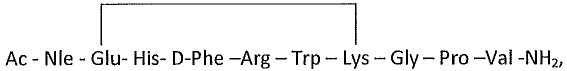
Ac - Nle - Glu- His- D-Phe –Arg – Trp – Lys – Gly – Pro –Val -NH2,

Ac – Nle- Glu – His - D-Phe – Arg - Trp - Lys-NH2,

Ac- Nle - Asp - His - D-Phe - Arg - Trp - Lys-NH2,

Ac- Nle – Asp - His - D-Phe - Arg - Trp - Orn-NH2,

Ac- Nle - Asp – His - D-Phe – Arg – Trp - Dab-NH2,

Ac- Nle – Asp - His - D-Phe - Arg - Trp -Dpr-NH2,

Ac- Ser – Tyr - Ser- Nle – Asp - His - D-Phe - Arg - Trp - Lys-Gly-Pro-Val-NH2,

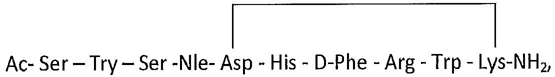
Ac- Ser – Try – Ser -Nle- Asp - His - D-Phe - Arg - Trp - Lys-NH2,

Ac-Tyr – Ser – Nle -Asp- His - D-Phe - Arg - Trp - Lys-NH2,

Ac- Ser - Nle - Asp - His - D-Phe - Arg – Trp - Lys-NH2,

Ac - Nle – Asp - His - D-Phe - Arg - Trp - Lys-Gly-NH2,

Ac- Nle - Asp - His - D-Phe - Arg - Trp - Lys-Gly-Pro-NH2,

Ac - Nle - Asp - His - D-Phe - Arg - Trp - Lys-Gly-Pro-Val-NH2, and

Ac - Ser - Nle - Asp - His - D-Phe - Arg - Trp - Lys-Gly-Pro-Val-NH2.

In another aspect, the alpha-MSH analogue is preferably selected from the group consisting of:
[D-Phe⁷]-α-MSH,
[Nle⁴, D-Phe⁷]-α-MSH,
[D-Ser¹, D-Phe⁷]-α-MSH,
[D-Tyr², D-Phe⁷]-α-MSH,
[D-Ser³, D-Phe⁷]-α-MSH,
[D-Met⁴, D-Phe⁷]-α-MSH,
[D-Glu⁵, D-Phe⁷]-α-MSH,
[D-His⁶, D-Phe⁷]-α-MSH,
[D-Phe⁷, D-Arg⁸]-α-MSH,
[D-Phe⁷, D-Trp⁹]-α-MSH,
[D-Phe⁷, D-Lys¹¹]-α-MSH,
[D-Phe⁷, D-Pro¹²]-α-MSH,
[D-Phe⁷, D-Val¹³]-α-MSH,
[D-Ser¹, Nle⁴, D-Phe⁷]-α-MSH,
[D-Tyr², Nle⁴, D-Phe⁷]-α-MSH,
[D-Ser³, Nle⁴, D-Phe⁷]-α-MSH,
[Nle⁴, D-Glu⁵, D-Phe⁷]-α-MSH,
[Nle⁴, D-His⁶, D-Phe⁷]-α-MSH,
[Nle⁴, D-Phe⁷, D-Arg⁸]-α-MSH,
[Nle⁴, D-Phe⁷, D-Trp⁹]-α-MSH,
[Nle⁴, D-Phe⁷, D-Lys¹¹]-α-MSH,
[Nle⁴, D-Phe⁷, D-Pro¹²]-α-MSH,
[Nle⁴, D-Phe⁷, D-Val¹³]-α-MSH, 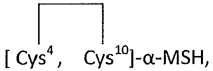
[ Cys⁴,   Cys¹⁰]-α-MSH,
 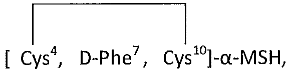
[ Cys⁴, D-Phe⁷, Cys¹⁰]-α-MSH,
 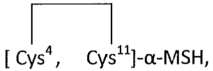
[ Cys⁴,     Cys¹¹]-α-MSH,
 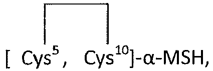
[ Cys⁵ , Cys¹⁰]-α-MSH,
 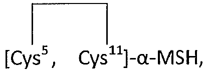
[Cys5, Cys11]-α-MSH,
 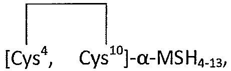
[Cys⁴,     Cys¹⁰]-α-MSH₄₋₁₃,
 [Cys4, Cys10]-α-MSH4-12,
[Nle⁴, D-Phe⁷]-α-MSH₄₋₁₀,
[Nle⁴, D-Phe⁷]-α-MSH₄₋₁₁,
[D-Phe⁷]-α-MSH₅₋₁₁,
[Nle⁴, D-Tyr⁷]-α-MSH₄₋₁₁,
[(pNO₂)D-Phe⁷]-α-MSH₄₋₁₁,
[Tyr⁴, D-Phe⁷]-α-MSH₄₋₁₀,
[Tyr⁴, D-Phe⁷]-α-MSH₄₋₁₁,
[Nle⁴]-α-MSH₄₋₁₁,
[Nle⁴, (pNO₂)D-Phe⁷]-α-MSH₄₋₁₁,
[Nle⁴, D-His⁶]-α-MSH₄₋₁₁,
[Nle⁴, D-His⁶, D-Phe⁷]-α-MSH₄₋₁₁,
[Nle⁴, D-Arg⁸]-α-MSH₄₋₁₁,
[Nle⁴, D-Trp⁹]-α-MSH₄₋₁₁,
[Nle⁴, D-Phe⁷, D-Trp⁹]-α-MSH₄₋₁₁,
[Nle⁴, D-Phe⁷]-α-MSH₄₋₉, and
[Nle⁴, D-Phe⁷, D-Trp⁹]-α-MSH₄₋₉.

Preferred alpha-MSH analogues thereof are selected from the group consisting of:
[Nle⁴, D-Phe⁷]-α-MSH₄₋₁₀,
[Nle⁴, D-Phe⁷]-α-MSH₄₋₁₁,
[Nle⁴, D-Phe⁷, D-Trp⁹]-α-MSH₄₋₁₁, and
[Nle⁴, D-Phe⁷]-α-MSH₄₋₉.

In another aspect, the alpha-MSH analogue is a cyclic peptide of formula (I):

Z-Xaa¹-Xaa²-Xaa³-Xaa⁴-Xaa⁵-Xaa⁶-Xaa⁷-Y (I)

or a pharmaceutically acceptable salt thereof, wherein:
Z is H or an N-terminal group wherein the N-terminal group is preferably a C₁ to C₁₇ acyl group, wherein the C₄ to C₁₇ comprises a linear or branched alkyl, cycloalkyl, alkylcycloalkyl, aryl or alkylaryl, a linear or branched C₁ to C17 alkyl, aryl, heteroaryl, alkene, alkenyl, or aralkyl chain or an N-acylated linear or branched C₁ to C₁₇ alkyl, aryl, heteroaryl, alkene, alkenyl, or aralkyl chain and more preferably is a C₁ to C₇ acyl group;
Xaa¹ is optionally present, and if present is from one to three L- or D-isomer amino acid residues, and preferably an amino with a side chain including a linear or branched alkyl, cycloalkyl, cycloheteroalkyl, aryl or heteroaryl, and more preferably is an L- or D-isomer of Nle;
Xaa² and Xaa⁶ are L- or D-isomer amino acids wherein the side chains thereof comprise a cyclic bridge, and, preferably, one of Xaa² and Xaa⁶ is an L- or D-isomer of Asp, hGlu or Glu and the other of Xaa² and Xaa⁶ is an L- or D-isomer of Lys, Orn, Dab or Dap or, in an alternative preferred aspect, Xaa² and Xaa⁶ are each Cys, D-Cys, Pen or D-Pen;
Xaa³ is L- or D-Pro, optionally substituted with hydroxyl, halogen, sulfonamide, alkyl, -O-alkyl, aryl, alkyl-aryl, alkyl-O-aryl, alkyl-O-alkyl-aryl, or -0-aryl, or Xaa³ is an L- or D-isomer of an amino acid with a side chain including at least one primary amine, secondary amine, alkyl, cycloalkyl, cycloheteroalkyl, aryl, heteroaryl, ether, sulfide, or carboxyl and preferably is an L- or D-isomer of His;
Xaa⁴ is an L- or D-isomer amino acid with a side chain including phenyl, naphthyl or pyridyl, optionally wherein the ring is substituted with one or more substituents independently selected from halo, (C₁-C₁₀)alkyl-halo, (C₁-C₁₀)alkyl, (C₁-C₁₀)alkoxy, (C₁-C₁₀)alkylthio, aryl, aryloxy, nitro, nitrile, sulfonamide, amino, monosubstituted amino, disubstituted amino, hydroxy, carboxy, and alkoxy-carbonyl, and is preferably D-Phe, optionally substituted with one or more substituents independently selected from halo, (C₁-C₁₀)alkyl-halo, (C₁-C₁₀)alkyl, (C₁₋C₁₀)alkoxy, (C₁-C₁₀)alkylthio, aryl, aryloxy, nitro, nitrile, sulfonamide, amino, monosubstituted amino, disubstituted amino, hydroxy, carboxy, and alkoxy-carbonyl;
Xaa⁵ is L- or D-Pro or an L- or D-isomer amino acid with a side chain including at least one primary amine, secondary amine, guanidine, urea, alkyl, cycloalkyl, cycloheteroalkyl, aryl, heteroaryl, or ether and preferably is an L- or D-isomer of Arg, Lys, Orn, Dab or Dap;
Xaa⁷ is optionally present, and if present is from one to three L- or D-isomer amino acid residues, and is preferably an amino acid with a side chain including at least one aryl or heteroaryl, optionally substituted with one or more ring substituents, and when one or more substituents are present, are the same or different and independently hydroxyl, halogen, sulfonamide, alkyl, -O-alkyl, aryl, or -O-aryl, and more preferably is an L- or D-isomer of Trp, Nal 1 or Nal 2; and
Y is a C-terminal group and in another aspect preferably a hydroxyl, an amide, or an amide substituted with one or two linear or branched C₁ to C₁₇ alkyl, cycloalkyl, aryl, alkyl cycloalkyl, aralkyl, heteroaryl, alkene, alkenyl, or aralkyl chains.

Preferred cyclic alpha-MSH analogues are Ac-Nle-cyclo(Glu-His-D-Phe-Arg-Dab)-Trp-NH₂ and Ac-Nle-cyclo(Glu-His-D-Phe-Arg-Dap)-Trp-NH₂.

According to this aspect and in addition to the above defined amino acids, the amino acids are defined in US2013/0296256 pages 5 and 6. Further, the terms "α,α-disubstituted amino acid", "N-substituted amino acid", "alkane", "alkene", "alkenyl", "alkyl", "alkyne", "aryl", "aralkyl", "aliphatic", "acyl", "acylated", "omega amino aliphatic chain", "heteroaryl", "amide", "imide", "amine", "nitrile", and "halogen" are defined on pages 6 and 7 thereof.

In a further aspect of the invention, the alpha-MSH analogue compound is a derivative of alpha-MSH which exhibits agonist activity for the melanocortin-1 receptor (MC1R), the receptor to which alpha-MSH binds to initiate the production of melanin within a melanocyte, wherein the alpha-MSH analogue comprises a quaternary ammonium group in the backbone. The quaternary ammonium group (that is attached to the alpha-MSH analogue) preferably has three substituents independently selected from methyl, ethyl and propyl. Preferably, the positively charged ammonium group is attached to the backbone of the alpha-MSH analogue with a -(CH2)ₙ-CO- intermediate group wherein n =1-4, preferably 1-3 and more preferably 1 or 3. For the purpose of the invention, the -(CH2)ₙ-CO- intermediate group is part of the quaternary ammonium group. Preferably, the alpha-MSH analogue (that is attached to the quaternary ammonium group) is a hexapeptide. Preferably, the hexapeptide comprises the following 6 units: Nle-Glu-His-D-Phe-X-Trp-NH₂ wherein X is selected from Arg, HomoArg and/or NorArg, preferably Arg or homoArg, providing the compound with added benefits including increased efficacy and efficient preparation, with high yield and/or high purity. Selection of homoArg provides further preferred benefits, including less susceptibility to degradation, increased efficacy, more stability and a high-yield preparation method.

The alpha-MSH analogue compound preferably has formula structure: R₁ R₂ R₃ N - (CH2)ₙ - CO - Nle - Glu - His- D-Phe - X - Trp - NH₂ wherein:
R₁, R₂ and R₃ are independently selected from methyl, ethyl, and propyl;
n is from 1-4; and
X is selected from Arg, norArg and homoArg,
or a pharmaceutically acceptable salt thereof.

A preferred compound is (C₂H₅)₃N-CH₂-CO-Nle-Glu-His-D-Phe-Arg-Trp-NH₂ or a pharmaceutically acceptable salt thereof. A preferred compound is (CH₃)₃N-(CH₂)₃-CO-Nle-Glu-His-D-Phe-Arg-Trp-NH₂ or a pharmaceutically acceptable salt thereof. A preferred compound is (C₂H₅)₃N-CH₂-CO-Nle-Glu-His-D-Phe-homoArg-Trp-NH₂ or a pharmaceutically acceptable salt thereof. A preferred compound is (CH₃)₃N-(CH₂)₃-CO-Nle-Glu-His-D-Phe-homoArg-Trp-NH₂ or a pharmaceutically acceptable salt thereof. A preferred compound is (C₂H₅)₃N-CH₂-CO-Nle-Glu-His-D-Phe-norArg-Trp-NH₂ or a pharmaceutically acceptable salt thereof. A preferred compound is (CH₃)₃N-(CH₂)₃-CO-Nle-Glu-His-D-Phe-norArg-Trp-NH₂ or a pharmaceutically acceptable salt thereof.

In a further aspect of the aspect, the alpha-MSH analogue compound has formula structure Ac - Nle - Glu - His- D-Phe - X - Trp - NH₂, wherein X is homoArg or norArg, or a pharmaceutically acceptable salt thereof.

According to the present invention, the most preferred alpha-MSH analogue is [Nle⁴, D-Phe⁷]-alpha-MSH. This preferred compound is sometimes referred to as NDP-MSH. It is also generically known as afamelanotide, which is available as an implant composition under the trademark SCENESSE^{®}.

Preferably, the alpha-MSH analogue is administered in a composition. Preferably, the composition is a controlled release composition, resulting in longer and/or more controlled exposure of the body to the drug. Most preferably, the composition is an implant. In one preferred embodiment, the alpha-MSH analogue is administered in a prolonged release composition such as described in US2008305152 (equivalent to WO2006/012667).

The composition preferably comprises at least 1mg of the alpha-MSH analogue, more preferably at least 4mg, most preferably at least 5mg, particularly preferred at least 10mg, and preferably at most 30mg, more preferably at most 25mg of the alpha-MSH analogue. Particularly preferred amounts are 20mg or 16mg of the alpha-MSH analogue of which 16mg of the alpha-MSH analogue is the most preferred.

Preferably, the composition comprises a controlled release composition. In one aspect according to the present invention, the implant (or rod) comprises a biodegradable polymer, wherein the alpha-MSH analogue is imbedded within the implant. In one aspect, the alpha-MSH analogue is encapsulated in an implant composed of poly-(lactide-co-glycolide), poly-(lactide), poly-(glycolide) or a mixture thereof. Lactide/glycolide polymers for drug-delivery compositions are typically made by melt polymerization through the ring opening of lactide and glycolide monomers. Some polymers are available with or without carboxylic acid end groups. When the end group of the poly-(lactide-co-glycolide), poly-(lactide), or poly-(glycolide) is not a carboxylic acid, for example, an ester, then the resultant polymer is referred to herein as blocked or capped. The unblocked polymer, conversely, has a terminal carboxylic group. In one aspect, linear lactide/glycolide polymers are used; however star polymers can be used as well. In certain aspects, high molecular weight polymers can be used for medical devices, for example, to meet strength requirements. The lactide portion of the polymer has an asymmetric carbon. Commercially racemic DL-, L-, and D-polymers are available. The L-polymers are more crystalline and resorb slower than DL- polymers. In addition to copolymers comprising glycolide and DL-lactide or L-lactide, copolymers of L-lactide and DL-lactide are available. Additionally, homo-polymers of lactide or glycolide are available. In the case when the biodegradable polymer is poly-(lactide-co-glycolide), poly-(lactide), or poly-(glycolide), the amount of lactide and glycolide in the polymer can vary. In one aspect, the biodegradable polymer contains 0 to 100 mole %, 40 to 100 mole %, 50 to 100 mole %, 60 to 100 mole %, 70 to 100 mole %, or 80 to 100 mole % lactide and from 0 to 100 mole %, 0 to 60 mole %, 10 to 40 mole %, 20 to 40 mole %, or 30 to 40 mole % glycolide, wherein the amount of lactide and glycolide is 100 mole %. In one aspect, the biodegradable polymer can be poly-(lactide), 85:15 poly-(lactide-co-glycolide), 75:25 poly-(lactide-co-glycolide), or 65:35 poly-lactide-co-glycolide) where the ratios are mole ratios. In one aspect, when the biodegradable polymer is poly-(lactide-co-glycolide), poly-(lactide), or poly-(glycolide), the polymer has an intrinsic viscosity of from 0.15 to 1.5 dL/g, 0.25 to 1.5 dL/g, 0.25 to 1.0 dL/g, 0.25 to 0.8 dL/g, 0.25 to 0.6 dL/g, or 0.25 to 0.4 dL/g as measured in chloroform at a concentration of 0.5 g/dL at 30°C.

The implant preferably comprises alpha-MSH analogue in an amount of from 5% to 60%, more preferably from 10% to 50%, most preferably from 15% to 40%, and in particularly preferred from 15% to 30% by weight of the implant. Preferred implants are described in US2008/0305152. A preferred implant comprising afamelanotide is available under the name of SCENESSE^{®} in Italian and Swiss markets.

Other pharmaceutically-acceptable components can be encapsulated or incorporated in the composition or in the implant in combination with the alpha-MSH analogue. For example, the pharmaceutically-acceptable component can include a fatty acid, a sugar, a salt, a watersoluble polymer such as polyethylene glycol, a protein, polysacharride, or carboxmethyl cellulose, a surfactant, a plasticizer, a high- or low- molecular- weight porosigen such as polymer or a salt or sugar, or a hydrophobic low- molecular-weight compound such as cholesterol or a wax.

### Example

XP is a disease that can be classified in eight complementation groups XP-A to XP-G as well as XP-variant (XP-V). The cause of XP is a mutation in the genetic coding, leading to synthesis of different, dysfunctional XP proteins of different structure. While the details of the functioning of these different XP proteins in subjects suffering from XP is not fully understood, the differences have a dramatic clinical effect on the DNA repair capability, leading to high DNA damage, skin cancer and death at a young age.

Despite the high level of DNA damage occurring in XP patients, we realized that XP patients have remaining -low or very low- DNA repair capability. We further realized that such residual activity however means that the complexes and pathways in XP patients are at least partially intact. Based on these insights and despite the XP protein in XP patients having a different structure with associated dysfunctionality, we realized not only that these different XP proteins are still being recruited for complexes used in the DNA repair pathways, but also - importantly- that administration of MC1R agonists according to the invention to XP patients further enhances such recruitment of factors -including the different XP protein- for these complexes. Further, we realized that alpha-MSH analogues of the invention, as MC1R agonists, often better associate with various allele variants of the MC1R receptor associated with DNA repair in comparison with natural alpha-MSH levels, rendering the subsequent factor recruitment and DNA repair in XP patients more effective. We also realized that exposure of XP patient to alpha-MSH analogue compounds over longer periods enhances factor recruitment and subsequent DNA repair even further. We conclude that use of alpha-MSH analogue compounds of the invention in XP patients leads to an improved DNA repair capability reducing the cancer risk for XP patients particularly when compared to alpha-MSH at natural levels.

The above shows that the alpha-MSH analogue compounds are suitable and can be successfully used for DNA repair in XP patients which the following clinical trial confirms: Subjects diagnosed with XP based on clinical symptoms and genotyping are assigned to one of the 8 XP subgroups, including at least XP-A, XP-B, XP-C, XP-E, XP-F and XP-V. Subjects are administered afamelanotide implants (SCENESSE^{®}) that maintained at a plasma levels higher than zero but not exceeding 10ng/ml. Implants were administered regularly every 5 days to maintain plasma levels not exceeding 10ng/ml for of periods of at least 10, 20, 30, 40, 50 and 60 days. Skin biopsies are taken according to the preferred method described in reference (literature reference 1): Dreze M, Calkins AS, Ga'licza J, Echelman DJ, Schnorenberg MR, et al. (2014) "Monitoring Repair of UV-induced 6-4-Photoproducts with a Purified DDB2 Protein Complex". PLoS ONE 9(1): e85896. doi:10.1371/journal.pone.0085896. Biopsies are taken before (comparison), during and after periods of afamelanotide exposure. The biopsies are used to determine the concentration of photoproducts and dimers, such as 6,4 CPD and 8-oxoguanine. A preferred method is described in reference (literature reference 2) McCready S. (2014), "An Immunoassay for Measuring Repair of UV Photoproducts". In: Keohavong P., Grant S. (eds) Molecular Toxicology Protocols. Methods in Molecular Biology (Methods and Protocols), vol 1105. Humana Press, Totowa, NJ.

The methods are useful for measuring repair in total genomic DNA, and are thought to be sufficiently sensitive to measure repair of damage induced by light and UV radiation. Repair of genomic material will be confirmed after administration of afamelanotide (compared to before administration) and show the positive and unexpected beneficial effects of afamelanotide on DNA repair in subjects suffering from XP.

## Claims

1. Alpha-MSH analogue compound with agonist activity for the MC1R receptor for use in enhancing DNA repair in a subject suffering from Xeroderma Pigmentosum (XP).

2. Alpha-MSH analogue compound for use according to claim 1 for enhancing UV-induced DNA damage repair capability in a subject suffering from XP an autosomal recessive genetic disorder in which repair of DNA is deficient.

3. Alpha-MSH analogue compound for use according to any one of claims 1-2, for at least partially reducing one or more of the symptoms of XP.

4. Alpha-MSH analogue compound for use according to any one of claims 1-3, wherein the compound has formula structure:
Ac - Nle - Glu - His- D-Phe - X - Trp - NH₂, wherein X is homoArg or norArg, or a pharmaceutically acceptable salt thereof.

5. Alpha-MSH analogue compound for use according to any one of claims 1-4, wherein the compound has formula structure:
R₁R₂R₃N-(CH2)ₙ-CO-Nle-Glu-His-D-Phe-X-Trp-NH₂ wherein:
R₁, R₂ and R₃ are independently selected from methyl, ethyl, and propyl;
n is from 1-4; and
X is selected from Arg, norArg and homoArg,
or a pharmaceutically acceptable salt thereof.

6. Alpha-MSH analogue compound for use according to any one of claims 1-4, wherein the compound is [Nle⁴, D-Phe⁷]-alpha-MSH, or a pharmaceutically acceptable salt thereof.

7. Alpha-MSH analogue compound for use according to any one of claims 1-6, wherein the subject suffers from XP selected from complementation group A (XP-A), complementation group B (XP-B), complementation group C (XP-C), complementation group E (XP-E) and complementation group F (XP-F) and variant type V (XPV).

8. Alpha-MSH analogue compound for use according to any one of claims 1-7, wherein the compound is administered in a composition providing plasma levels of the compound of less than 10ng/ml for at least 1 day.

9. Alpha-MSH analogue compound for use according to any one of claims 1-8, wherein the compound is administered in an extended release composition.

10. Alpha-MSH analogue compound for use according to any one of claims 6-9, wherein the composition comprises from 4 to 20mg of the alpha-MSH analogue.

11. Alpha-MSH analogue compound for use according to any one of claims 8-10, wherein the composition is administered with a dosing frequency of between 5 to 15 days.

12. Alpha-MSH analogue compound for use according to any one of claims 8-11, wherein the composition is administered at least 3 times consecutively to the subject.

13. Alpha-MSH analogue compound for use according to any one of claims 1-12, wherein the subject is a human subject.

14. Alpha-MSH analogue compound for use according to any one of claims 1-13, wherein the subject suffers XP-C.

15. Alpha-MSH analogue compound for use according to any one of claims 1-13, wherein the subject suffers XP-V.

## Patentansprüche

1. Alpha-MSH-Analogverbindung mit Agonistenaktivität für den MC1R-Rezeptor zur Verwendung in der Verbesserung der DNA-Reparatur bei einem Individuum, das an Xeroderma pigmentosum (XP) leidet.

2. Alpha-MSH-Analogverbindung zur Verwendung gemäß Anspruch 1 für die Verbesserung der Fähigkeit zur Reparatur UV-induzierter DNA-Schäden bei einem Individuum, das an XP leidet, einem autosomalen rezessiven Gendefekt, bei dem die DNA-Reparatur mangelhaft ist.

3. Alpha-MSH-Analogverbindung zur Verwendung gemäß einem beliebigen der Ansprüche 1-2, um ein oder mehrere Symptome von XP zumindest teilweise abzuschwächen.

4. Alpha-MSH-Analogverbindung zur Verwendung gemäß einem beliebigen der Ansprüche 1-3, wobei die Verbindung folgende Formelstruktur hat:
Ac-Nle-Glu-His-D-Phe-X-Trp-NH₂, worin X homoArg oder norArg ist, oder ein pharmazeutisch verträgliches Salz davon.

5. Alpha-MSH-Analogverbindung zur Verwendung gemäß einem beliebigen der Ansprüche 1-4, wobei die Verbindung folgende Formelstruktur hat:
R₁R₂R₃N-(CH2)ₙ-CO-Nle-Glu-His-D-Phe-X-Trp-NH₂, worin
R₁, R₂ und R₃ unabhängig gewählt sind aus Methyl, Ethyl und Propyl,
n von 1-4 ist und
X gewählt ist aus Arg, norArg und homoArg;
oder ein pharmazeutisch verträgliches Salz davon.

6. Alpha-MSH-Analogverbindung zur Verwendung gemäß einem beliebigen der Ansprüche 1-4, wobei die Verbindung [Nle⁴, D-Phe⁻⁷]-alpha-MSH ist, oder ein pharmazeutisch verträgliches Salz davon.

7. Alpha-MSH-Analogverbindung zur Verwendung gemäß einem beliebigen der Ansprüche 1-6, wobei das Individuum an XP leidet, gewählt aus Komplementationsgruppe A (XP-A), Komplementationsgruppe B (XP-B), Komplementationsgruppe C (XP-C), Komplementationsgruppe E (XP-E) und Komplementationsgruppe F (XP-F) und Variante Typ V (XPV).

8. Alpha-MSH-Analogverbindung zur Verwendung gemäß einem beliebigen der Ansprüche 1-7, wobei die Verbindung in einer Zusammensetzung verabreicht wird, die für Plasmaspiegel der Verbindung von weniger als 10ng/ml über mindestens 1 Tag sorgt.

9. Alpha-MSH-Analogverbindung zur Verwendung gemäß einem beliebigen der Ansprüche 1-8, wobei die Verbindung in einer Zusammensetzung mit erweiterter Freisetzung verabreicht wird.

10. Alpha-MSH-Analogverbindung zur Verwendung gemäß einem beliebigen der Ansprüche 6-9, wobei die Zusammensetzung 4 bis 20mg des Alpha-MSH-Analogons umfasst.

11. Alpha-MSH-Analogverbindung zur Verwendung gemäß einem beliebigen der Ansprüche 8-10, wobei die Zusammensetzung mit einer Dosierfrequenz zwischen 5 und 15 Tagen verabreicht wird.

12. Alpha-MSH-Analogverbindung zur Verwendung gemäß einem beliebigen der Ansprüche 8-11, wobei die Verbindung dem Individuum mindestens 3-mal hintereinander verabreicht wird.

13. Alpha-MSH-Analogverbindung zur Verwendung gemäß einem beliebigen der Ansprüche 1-12, wobei das Individuum ein menschliches Individuum ist.

14. Alpha-MSH-Analogverbindung zur Verwendung gemäß einem beliebigen der Ansprüche 1-13, wobei das Individuum an XP-C leidet.

15. Alpha-MSH-Analogverbindung zur Verwendung gemäß einem beliebigen der Ansprüche 1-13, wobei das Individuum an XP-V leidet.

## Revendications

1. Composé analogue d'alpha-MSH avec une activité agoniste pour le récepteur MC1R destiné à être utilisé dans l'augmentation de la réparation d'ADN chez un sujet souffrant de Xeroderma pigmentosum (XP).

2. Composé analogue d'alpha-MSH destiné à être utilisé selon la revendication 1 pour augmenter la capacité de réparation de dommage d'ADN induite par les UV chez un sujet souffrant de XP un trouble génétique récessif autosomal dans lequel la réparation de l'ADN est déficiente.

3. Composé analogue d'alpha-MSH destiné à être utilisé selon l'une quelconque des revendications 1 à 2, pour au moins partiellement réduire un ou plusieurs des symptômes de XP.

4. Composé analogue d'alpha-MSH destiné à être utilisé selon l'une quelconque des revendications 1 à 3, dans lequel le composé a la structure de formule :
Ac-Nle-Glu-His-D-Phe-X-Trp-NH₂, X étant l'homoArg ou la norArg ou un de ses sels pharmaceutiquement acceptables.

5. Composé analogue d'alpha-MSH destiné à être utilisé selon l'une quelconque des revendications 1 à 4, dans lequel le composé a la structure de formule :
R₁R₂R₃N-(CH2)ₙ-CO-Nle-Glu-His-D-Phe-X-Trp-NH₂ :
R₁, R₂ et R₃ étant indépendamment choisis parmi un groupe méthyle, éthyle et propyle ;
n valant de 1 à 4 ; et
X étant choisi parmi Arg, norArg et homoArg,
ou un de ses sels pharmaceutiquement acceptables.

6. Composé analogue d'alpha-MSH destiné à être utilisé selon l'une quelconque des revendications 1 à 4, dans lequel le composé est [Nle⁴, D-Phe⁷]-alpha-MSH ou un de ses sels pharmaceutiquement acceptables.

7. Composé analogue d'alpha-MSH destiné à être utilisé selon l'une quelconque des revendications 1 à 6, dans lequel le sujet souffre de XP choisi parmi le groupe A de complémentation (XP-A), le groupe B de complémentation (XP-B), le groupe C de complémentation (XP-C), le groupe E de complémentation (XP-E) et le groupe F de complémentation (XP-F) et le variant de type V (XPV).

8. Composé analogue d'alpha-MSH destiné à être utilisé selon l'une quelconque des revendications 1 à 7, dans lequel le composé est administré dans une composition fournissant des niveaux de plasma du composé de moins de 10 ng/ml durant au moins 1 jour.

9. Composé analogue d'alpha-MSH destiné à être utilisé selon l'une quelconque des revendications 1 à 8, dans lequel le composé est administré dans une composition à libération prolongée.

10. Composé analogue d'alpha-MSH destiné à être utilisé selon l'une quelconque des revendications 6 à 9, dans lequel la composition comprend de 4 à 20 mg de l'analogue d'alpha-MSH.

11. Composé analogue d'alpha-MSH destiné à être utilisé selon l'une quelconque des revendications 8 à 10, dans lequel la composition est administrée avec une fréquence de dosage d'entre 5 à 15 jours.

12. Composé analogue d'alpha-MSH destiné à être utilisé selon l'une quelconque des revendications 8 à 11, dans lequel la composition est administrée au moins 3 fois consécutivement au sujet.

13. Composé analogue d'alpha-MSH destiné à être utilisé selon l'une quelconque des revendications 1 à 12, dans lequel le sujet est un sujet humain.

14. Composé analogue d'alpha-MSH destiné à être utilisé selon l'une quelconque des revendications 1 à 13, dans lequel le sujet souffre de XP-C.

15. Composé analogue d'alpha-MSH destiné à être utilisé selon l'une quelconque des revendications 1 à 13, dans lequel le sujet souffre de XP-V.
